# EUROPÄISCHE PATENTSCHRIFT

(11) **EP 1 448 267 B1**
(45) Veröffentlichungstag und Bekanntmachung des Hinweises auf die Patenterteilung: **11.04.2007**
(21) Anmeldenummer: 02777323.3
(22) Anmeldetag: 23.10.2002
(51) Int. Cl.: A61N 5/10, G21K 1/04

(54) **KOLLIMATOR FÜR ENERGIEREICHE STRAHLUNG UND PROGRAMM ZUR STEUERUNG DES KOLLIMATORS**
COLLIMATOR FOR HIGH-ENERGY RADIATION AND PROGRAM FOR CONTROLLING SAID COLLIMATOR
COLLIMATEUR DESTINE A DES RAYONS A GRANDE ENERGIE ET PROGRAMME DE COMMANDE DE CE COLLIMATEUR

(30) Priorität: 23.11.2001 DE 10157523
(43) Veröffentlichungstag der Anmeldung: 25.08.2004
(73) Patentinhaber: Deutsches Krebsforschungszentrum Stiftung des öffentlichen Rechts, 69120 Heidelberg (DE)
(72) Erfinder: PASTYR, Otto, 69181 St. Ilgen (DE); ECHNER, Gernot, 69257 Wiesenbach (DE); SCHLEGEL, Wolfgang, 69118 Heidelberg (DE); HARTMANN, Günther, 69121 Heidelberg (DE)
(74) Vertreter: Weber, Walter
(86) Internationale Anmeldenummer: PCT/EP2002/011812
(87) Internationale Veröffentlichungsnummer: WO 2003/043698

(56) Entgegenhaltungen:
- EP-A- 0 382 560
- DE-A- 19 950 794
- DE-C- 19 905 823
- US-A- 5 332 908
- US-A1- 2001 043 669

## Beschreibung

Die Erfindung betrifft einen Kollimator zum Begrenzen eines Bündels energiereicher Strahlen, das von einer im wesentlichen punktförmigen Strahlungsquelle ausgehend auf ein Behandlungsobjekt gerichtet ist und insbesondere der stereotaktischen Konformationsbestrahlung von Tumoren dient, wobei der Kollimator derart ausgebildet ist, daß ein unregelmäßiges Behandlungsobjekt mittels Strahlen abscannbar ist, die durch eine Kollimatoröffnung begrenzt sind.

Kollimatoren dienen dazu, daß kompliziert geformte Zielvolumina konform und hochdosiert bestrahlt werden können. Bekannt sind verschiedene Arten von Kollimatoren, wobei jedoch eine hohe Genauigkeit, insbesondere eine exakte Begrenzung der Bestrahlungsfelder regelmäßig nur mit hohem Zeitaufwand und/oder großem technischen Aufwand erzielbar ist.

Zeitaufwendig sind insbesondere Kompensatoren, da für jede zu bestrahlende Fläche eine Form gefertigt werden muß und die Formen zwischen den einzelnen Einstrahlrichtungen an den Bestrahlungsgeräten gewechselt werden müssen.

Um diesen Zeitaufwand zu verringern, werden heutzutage meist Multileafkollimatoren eingesetzt. Ein solcher ist beispielsweise aus der DE 199 05 823 C1 bekannt. Die Formbildung erfolgt dort durch eine Vielzahl von Blendenblättern (Leafs), wobei jedem Leaf ein Motor, eine Wegerfassung und eine Steuerung der Ausrichtung der Vorderkante des Leafs nach dem Strahlengang zugeordnet ist. Dieser Aufwand muß für eine Größenordnung von 80 bis 120 Leaves betrieben werden, wobei für jede zu bestrahlende Fläche und auch zur Erzeugung verschiedener Bestrahlungsintensitäten die Leaves verstellt werden müssen.

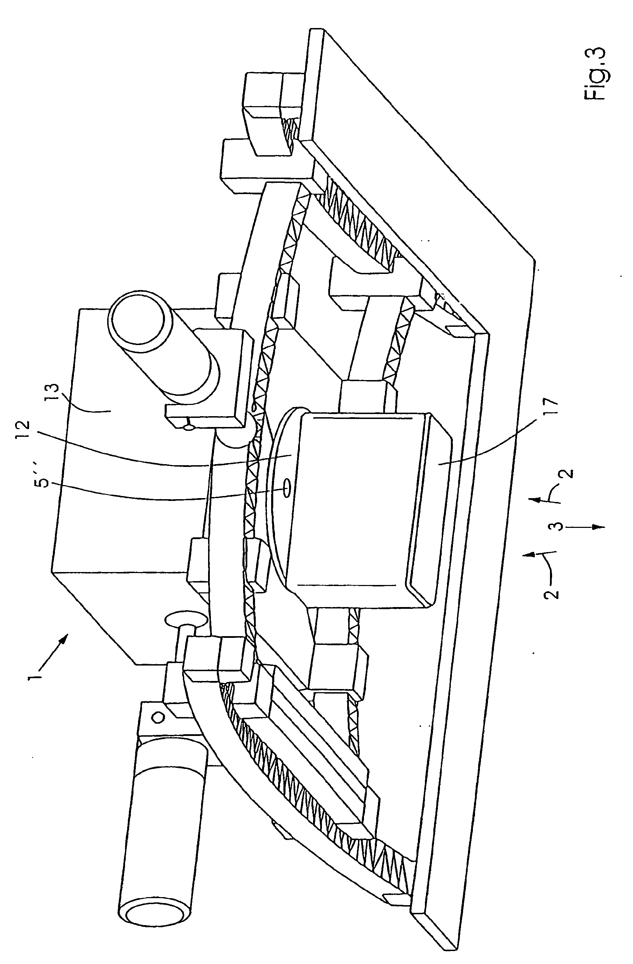

Ein Kollimator der eingangs genannten Art ist aus der DE 199 22 656 A1 bekannt. Bei dieser Vorrichtung wird allerdings ein Elektronenstrahl mittels eines Magnetfeldes ausgelenkt und dadurch gescannt, wobei der gescannte Strahl danach in einen Photonenstrahl zur Bestrahlung umgewandelt wird. Der Kollimator ist dabei eine Weiterbildung dieses Gegenstandes zur Ausbildung eines noch exakteren Bestrahlungspunktes. Wenn der Kollimator auch als einfache Öffnung ausgebildet ist, so ist der Gesamtaufbau des Geräts dennoch kompliziert, insbesondere bei der Ausführungsvariante der Fig. 3, bei der die Kollimatoröffnung synchron mit dem gescannten Strahl bewegt werden muß.

Vor allem besteht der Nachteil dieses Kollimators darin, daß die Genauigkeit, mit der die Begrenzungen von Bestrahlungsfeldern nachgebildet werden können, von der Größe des Bestrahlungspunktes, der mit Hilfe der bei diesem Kollimator festgelegten Kollimatoröffnung gebildet wird, abhängt. Große Bestrahlungspunkte mittels großer Kollimatoröffnungen führen zu einem schnelleren Abscannen, aber zu einer größeren Ungenauigkeit, umgekehrt verlangsamen kleine Bestrahlungspunkte das Abscannen, führen aber zu einer genaueren Nachbildung der Begrenzungen von Bestrahlungsfeldern.

Der Erfindung liegt daher die Aufgabe zugrunde, die Konturen der zu bestrahlenden Behandlungsobjekte mit geringerem Aufwand aber hoher Genauigkeit und insbesondere mit einer exakten Begrenzung der Bestrahlungsfelder zu erzeugen.

Eine zusätzliche Aufgabe besteht darin, die Intensität der Bestrahlung für verschiedene Teilbereiche der Behandlungsobjekte schnell und einfach variieren zu können.

Die Aufgabe wird erfindungsgemäß dadurch gelöst, daß er mehrere Kollimatoröffnungen in verschiedenen Größen aufweist, wobei wahlweise eine davon auf einer kugeloberflächenförmigen Bahn allseitig verschiebbar und mit ihrer Mittelachse auf die Strahlungsquelle ausgerichtet ist und wobei die anderen Kollimatoröffnungen von den Strahlen abgeschirmt sind, und daß er eine Steuerung aufweist, die so ausgebildet ist, daß sie auf die Antriebe einer Antriebseinrichtung derart wirkt, daß für das Abscannen großer Bestrahlungsflächen des Behandlungsobjekts große Kollimatoröffnungen und für die exakte Begrenzung am Rande der Bestrahlungsflächen des Behandlungsobjekts, insbesondere bei unregelmäßigen Konturen, kleine Kollimatoröffnungen eingesetzt werden.

Bezüglich des Programms wird die Aufgabe dadurch gelöst, daß es derart ausgebildet ist, daß es mittels eines Rechners die Antriebseinrichtung derart steuert, daß ein Behandlungsobjekt entsprechend der vorbestimmten Konturen bestrahlbar ist, wobei es durch Betätigung des weiteren Antriebs die für die Ausbildung der Kontur optimale Größe einer Kollimatoröffnung auswählt und die Scannvorgänge, die mit den verschiedenen Kollimatoröffnungen ausgeführt werden, zur Erzielung der geforderten Bestrahlung aufeinander abstimmt.

Der erfindungsgemäße Gegenstand ermöglicht eine wesentliche Verkürzung des Scannvorgangs, da die Begrenzungslinien in sehr hoher Genauigkeit durch die Bestrahlung mittels einer sehr kleinen Kollimatoröffnung abgescannt werden können und dabei eine Restfläche lassen, welche mit einer mittleren oder einer größeren Kollimatoröffnung in wesentlich kürzerer Zeit abgescannt werden kann.

Der erfindungsgemäße Gegenstand eröffnet auch die Möglichkeit zur Lösung der zusätzlichen Aufgabe, nämlich die Steuerung derart auszubilden, daß die Verweilzeit der Strahlen, insbesondere die Scanngeschwindigkeit, mittels der vorgenannten Antriebseinrichtung steuerbar ist. Insbesondere wird die zusätzliche Aufgabe durch ein Programm gelöst, durch das ein mit ihm geladener Rechner dazu eingerichtet werden kann, mittels der vorgenannten Antriebseinrichtung die Verweilzeit, insbesondere die Scanngeschwindigkeit, zur Erzielung unterschiedlicher Bestrahlungsintensitäten zu steuern.

Die vorgeschlagene technische Lösung ist mit wesentlich geringerem technischem Aufwand wie bei den eingangs genannten Multileaflcollimatoren verbunden, da Konturen von Bestrahlungsfeldern mit Hilfe einer Antriebseinrichtung erzeugt werden können, die aus zwei, maximal aus drei Einzelantrieben aufgebaut ist. Entsprechend verringert sich der Aufwand für die Wegerfassung. Auch eine Vorderkanteneinstellung von Leaves ist nicht erforderlich. Entsprechend der Größe der Kollimatoröffnung kann eine beliebig kleine oder große Bestrahlungsfläche erzeugt werden, die nach dem Scanning-Prinzip über beliebig kompliziert geformte Flächen geführt wird. Dabei ist es auch möglich in einer zu bestrahlenden Fläche Inseln auszusparen, was bei den eine Fläche begrenzenden Kollimatoren nicht möglich ist. Dasselbe gilt auch für Flächen unterschiedlicher Bestrahlungsintensität, die beliebig angeordnet sein können. Dadurch ist es möglich ein Behandlungsobjekt bereichsbezogen mit unterschiedlichen Bestrahlungsintensitäten zu beaufschlagen. Auch dies ist möglich, ohne daß bezüglich der Formgebungsmöglichkeiten Grenzen gesetzt sind.

Vorzugsweise wird die mindestens eine Kollimatoröffnung derart ausgestaltet, daß sie in Richtung des Strahlengangs, also entsprechend konisch verlaufende Begrenzungen aufweist. Auf diese Weise wird eine exakte Begrenzung des zu bestrahlenden Feldes erzielt und jegliches Auftreten eines Halbschattens vermieden. Ein solcher tritt auf, wenn bei den Begrenzungen der Kollimatoren des Standes der Technik Bereiche vorhanden waren, in denen die Strahlen nicht durch die gesamte Materialstärke des Kollimators abgeschirmt wurden. Dieses Problem trat insbesondere bei den Multileafkollimatoren auf, da bei einer Verstellung der Leaves die Strahlen in jeder Leafstellung in einem andern Winkel verliefen. Bei dem eingangs genannten Multileafkollimator wurde dieses Problem dadurch gelöst, daß die Vorderkanten entsprechend der Leafeinstellung einen bestimmten Neigungswinkel aufweisen. Dies erforderte jedoch eine komplizierte Stellbewegung an allen vorhandenen Leaves. Die oben genannte Ausgestaltung der Erfindung löst dieses Problem auf sehr einfache Weise, da die das Bestrahlungsfeld abscannende Kollimatoröffnung sich auf einer kugeloberflächenförmig ausgebildeten Bahn bewegt und im wesentlichen immer auf die im wesentlichen punktförmige Strahlungsquelle gerichtet ist, so daß die Begrenzungen auch immer in Richtung des Strahlengangs verlaufen und deshalb ein Halbschatten nicht entstehen kann. Die mindestens eine Kollimatoröffnung kann dabei eine beliebige Form aufweisen, sie kann beispielsweise rund oder viereckig sein. Für die jeweils gewählte Form muß jedoch beim Scannvorgang berücksichtigt werden, daß die Bestrahlungszeit für jeden Punkt der Bestrahlungszeit entspricht, die der Behandlungsplan vorgibt.

Der Kollimator ist vorzugsweise derart ausgestaltet, daß mehrere, vorzugsweise drei Kollimatoröffnungen um eine gedachte Kreisbahn drehbar angeordnet sind. Der Vorteil dieser Ausgestaltung besteht darin, daß mittels einer drittel Kreisbewegung von einer Kollimatoröffnung auf eine der beiden anderen Öffnungsgrößen umgeschaltet werden kann, ohne daß dabei eine dazwischenliegende Kollimatoröffnung übersprungen werden muß. Dadurch kann der Umschaltvorgang auch während der Beaufschlagung mit dem Strahl vorgenommen werden, ohne daß während des Umschaltvorgangs Bestrahlungsintensitäten in nicht kalkulierter Form auf das Behandlungsobjekt treffen. Die Kreisbahn ist dann zweckmäßigerweise derart eingerichtet, daß die Kollimatoröffnung, die sich in der Arbeitsposition befindet, immer in erfindungsgemäßer Weise ausgerichtet ist und die anderen Kollimatoröffnungen abgeschirmt sind. Durch die drittel Kreisbewegung tritt die gewünschte andere Kollimatoröffnung an die Stelle der vorherigen, ohne daß eine Ausrichtung vorgenommen werden muß, da sich die Mittelpunkte der Kollimatoröffnungen alle auf der gedachten Kreisbahn befinden. Auch die Ausrichtung der Mittelachse einer Kollimatoröffnung zur Strahlungsquelle ist immer dann gewährleistet, wenn sich diese in der Arbeitsposition befindet.

Vorzugsweise werden die vorgenannten Ausgestaltungen derart ausgeführt, daß sich die Kollimatoröffnungen auf einem zylindrischen Teil aus abschirmendem Material befinden, das durch einen revolverähnlichen Mechanismus drehbar und mindestens in den Arbeitspositionen festlegbar ist. Dabei kann das zylindrische Teil in einem Abschirmungsblock drehbar gelagert sein.

Vorzugsweise ist vorgesehen, daß die mindestens eine Kollimatoröffnung gegen mindestens eine Kollimatoröffnung einer anderen Größe austauschbar ist. Dies kann in vorbeschriebener Weise der Fall sein oder durch eine Ausgestaltung, bei der nur eine Kollimatoröffnung vorgesehen ist, welche dann durch einen Austausch durch eine Kollimatoröffnung einer anderen Größe ersetzt werden kann. In beiden Ausgestaltungsvarianten ist es möglich ein beliebig großes Sortiment von Kollimatoröffnungen für alle möglichen Anwendungsfälle bereitzustellen. Sind die Kollimatoröffnungen in das zylindrische Teil eingefügt, so können mehrere zylindrische Teile mit verschiedenen Kollimatoröffnungsgrößen vorgesehen sein. Ein einfacherer Austausch der Kollimatoröffnungen kann jedoch bei allen Ausgestaltungen dadurch vorgenommen werden, daß ein Satz von Kollimatoröffnungen verschiedener Größen mittels austauschbarer Hülsen aus abschirmendem Material in den Kollimator einfügbar sind. Dabei haben die Hülsen äußerlich vorzugsweise dieselbe Kontur und enthalten lediglich Kollimatoröffnungen verschiedener Größen, so daß die Kollimatoröffnungsgrößen durch einfachen Hülsenaustausch variiert werden können.

Da beim Aneinanderfügen von abschirmenden Bauteilen selbst bei hoher Fertigungspräzision ein gewisser, wenn auch noch so geringer Spalt unvermeidbar ist, ist es zweckmäßig, wenn die Aneinaderfügungen abschirmender Bauteile keine parallel zum Strahlengang verlaufende durchgehende Berührungsflächen aufweisen. Dies kann dadurch erfolgen, daß sie in einem anderen Winkel verlaufen als die abzuschirmenden Strahlen. Vorzugsweise wird jedoch vorgesehen, daß die Berührungsflächen der Bauteile Absätze aufweisen, so daß es auch dann nicht zu einer Leckstrahlung durch Spalte kommen kann, wenn es durch die Scannbewegung doch zu einer, wenn auch partiellen parallelen Ausrichtung der Aneinanderfügungen zum Strahlengang kommen sollte. Dies gilt für alle Bauteile, welche sich im Strahlengang befinden, wie dem Abschirmungsblock mit dem zylindrischen Teil jedoch auch den austauschbaren Hülsen mit den Kollimatoröffnungen.

Bei der Ausgestaltung mit mehreren Kollimatoröffnungen muß, wie bereits erwähnt, eine Abschirmung der nicht in der Arbeitsposition befindlichen Kollimatoröffnungen vorgenommen werden. Vorzugsweise ist vorgesehen, daß der Abschirmung dieser Kollimatoröffnungen ein weiterer Abschirmungsblock dient. Dieser weitere Abschirmungsblock kann an dem bereits erwähnten Abschirmungsblock angeordnet sein oder es ist möglich, daß der weitere Abschirmungsblock feststehend am Kollimator angeordnet ist und seine Ausdehnung derart bemessen ist, daß die sich nicht in der Arbeitsposition befindenden Kollimatoröffnungen bei jeder Verschiebung der in der Arbeitsposition liegenden Kollimatoröffnung - und damit in der Regel auch des Abschirmungsblocks - gegenüber den Strahlen im Schatten des weiteren Abschirmungsblocks liegen. Diese Ausgestaltung hat den Vorteil, daß nur der erstgenannte Abschirmungsblock, jedoch nicht der weitere Abschirmungsblock bewegt werden muß, um den Scannvorgang zu erzielen. Dies ist deshalb von Bedeutung, weil die Abschirmungen sehr schwere Teile sind, da sie aus dickem abschirmenden Metall bestehen. Je weniger Gewicht jedoch verschoben werden muß, je weniger träge sind die Verschiebungen und damit der Scannvorgang und je geringer ist der Aufwand für die äußerst präzise vorzunehmenden Verschiebungen.

Die kugeloberflächenförmige Bahn läßt sich auf verschiedene Weise realisieren. Beispielsweise könnte der Abschirmungsblock kalottenförmig ausgebildet sein und auf einem Kalottenring bewegbar sein, der die in der Arbeitsposition befindliche Kollimatoröffnung nicht abdeckt. Vorzugsweise wird jedoch vorgesehen, daß die kugeloberflächenförmige Bahn durch zwei senkrecht zueinander verlaufende Gleitschienen gebildet wird, auf denen der Abschirmungsblock verschiebbar ist. Auch hier gibt es verschiedene Möglichkeiten der Ausgestaltung, vorzugsweise wird vorgesehen, daß die Gleitschienen durch Schienenpaare gebildet sind, wobei ein erstes Schienenpaar fest mit dem am Bestrahlungsgerät angeordneten Kollimatorgehäuse verbunden ist, das zweite Schienenpaar einem ersten Gleitschlitten zugeordnet ist, der auf dem ersten Schienenpaar läuft und daß der Abschirmblock auf einem zweiten Gleitschlitten angeordnet ist, der auf dem zweiten Schienenpaar läuft, das vorzugsweise senkrecht zum ersten Schienenpaar angeordnet ist. Auf diese Weise werden stabile Lagerungen mit definierten Bewegungsmöglichkeiten erreicht, so daß jeder Punkt auf der kugeloberflächenförmigen Bahn erreicht werden kann, beispielsweise durch eine Scannbewegung in einem x/y-Koordinatensystem, das dieser Kugelfläche zugeordnet sein kann.

Die Antriebseinrichtung wird vorzugsweise derart ausgestaltet, daß zur Verschiebung der mindestens einen Kollimatoröffnung auf der kugeloberflächenförmigen Bahn zwei Antriebe vorgesehen sind. Derartige Antriebe in einer x- und einer y-Richtung ermöglichen die Ansteuerung beliebiger Koordinaten, die mittels eines entsprechenden Programms ohne weiteres ansteuerbar sind, so daß die Bestrahlung in der Art vorgenommen werden kann, wie dies der Behandlungsplan vorsieht. Bei der vorgenannten konkreten Ausgestaltung werden die Antriebe zur Bewegung der Gleitschlitten an diesen angeordnet. Die Abstützung der Antriebe kann derart erfolgen, daß der Antrieb des ersten Gleitschlittens am Kollimatorgehäuse abgestützt ist und der zweite Antrieb des zweiten Gleitschlittens am ersten Gleitschlitten. Wesentlich ist, daß die Antriebe spielfrei sind und verschleißarm hohe Kräfte bewegen können, da das Abschirmungsmaterial, wie bereits erwähnt, ein hohes Gewicht aufweist. Zu diesem Zweck können die Antriebe beispielsweise mit Kugelgewindespindeln oder mit Rollengewindespindeln ausgestattet sein.

Für einen Kollimator mit mehreren Kollimatoröffnungen ist es zweckmäßig, wenn die Antriebseinrichtungen einen weiteren Antrieb zur Verbringung der jeweils gewünschten Kollimatoröffnung in die Arbeitsposition aufweist. Dies kann beispielsweise derart ausgestaltet sein, daß der weitere Antrieb auf dem Abschirmungsblock angeordnet ist, um das zylindrische Teil in die Arbeitspositionen der Kollimatoröffnungen zu drehen. Auf diese Weise läßt sich auch der Wechsel der Kollimatoröffnungen automatisieren und es ist möglich, alle Arbeitsschritte über eine Computersteuerung vorzunehmen.

Die Antriebseinrichtung kann auf unterschiedliche Weise ausgestaltet sein. Die Antriebe sollten zur Verbindung mit der Steuerung lediglich elektrisch ansteuerbar sein. Beispielsweise kann es sich um elektromotorische Antriebe, wie Schrittmotoren, Linearmotoren usw. handeln. Es ist jedoch auch möglich pneumatische oder hydraulische Antriebe mit elektrischen Steuerungen oder als Hybridantriebe ausgestaltet einzusetzen oder eine Kombination aus verschiedenen Antrieben.

Eine Steuerung der Intensität der Bestrahlung kann dadurch erzielt werden, daß die Steuerung so ausgebildet ist, daß die Verweilzeit der Strahlen mittels der Antriebseinrichtung steuerbar ist. Vorzugsweise ist die Verweilzeit durch die Scanngeschwindigkeit der Strahlen erzielbar, mit der diese das Behandlungsobjekt überstreichen.

Bei der Steuerung handelt es sich vorzugsweise um einen mittels eines entsprechenden Programms geladenen Rechner, um durch Veränderung der Software die Arbeitsweise einfach ändern zu können. Um sämtliche vorgenommenen Stellvorgänge verifizieren zu können, ist es zweckmäßig, wenn der Kollimator mit Positionserfassungseinrichtungen für die Stellbewegungen ausgestattet ist. Da die Steuerung zweckmäßigerweise derart ausgebildet ist, daß der Scannvorgang mittels eines vorgegebenen Behandlungsplans erfolgt, eröffnen die Positionserfassungseinrichtungen die Möglichkeit, daß eine Verifikation und gegebenenfalls eine Korrektur der Stellbewegungen vorgenommen werden kann.

Zur Steuerung werden heutzutage in der Regel Universalrechner eingesetzt, die für die konkreten Steuervorgänge mit einem entsprechenden Programm geladen sind. Deshalb ist ein Programm zur Steuerung des erfindungsgemäßen Kollimators ein weiterer Gegenstand dieser Erfindung. Dieses Programm wird zweckmäßigerweise derart ausgebildet, daß es mittels eines Rechners die Antriebseinrichtung derart steuert, daß ein Behandlungsobjekt entsprechend der vorbestimmten Konturen bestrahlbar ist, wobei es durch Betätigung des weiteren Antriebs die für die Ausbildung der Kontur optimale Größe einer Kollimatoröffnung auswählt und die Scannvorgänge, die mit den verschiedenen Kollimatoröffnungen ausgeführt werden, zur Erzielung der geforderten Bestrahlung aufeinander abstimmt.

Um das Behandlungsobjekt in seinen einzelnen Teilbereichen mit verschiedenen Bestrahlungsintensitäten beaufschlagen zu können, ist es zweckmäßig, wenn das Programm derart ausgebildet ist, daß es die Bestrahlungsintensität mittels der Verweilzeit durch eine entsprechende Steuerung der Antriebseinrichtung variieren kann.

Eine zweckmäßige Arbeitsweise des Programms kann darin bestehen, daß es die jeweilige Behandlungsfläche für ein Behandlungsobjekt in Teilfeldsegmente unterteilt und nach diesen Teilfeldsegmenten die Bestrahlung mittels des Rechners steuert. Da ein Behandlungsobjekt in der Regel aus verschiedenen Richtungen bestrahlt wird, ist vorgesehen, daß das Programm die Unterteilung in Teilfeldsegmente für die Bestrahlungsflächen für verschiedene Bestrahlungsrichtungen vornimmt.

Grundlage für eine Bestrahlung ist in der Regel eine vorher vorgenommene Bilderfassung des Behandlungsobjekts. Deshalb sollte das Programm derart ausgebildet sein, daß es die Bestimmung der Bestrahlungsflächen auf der Grundlage der Erfassung der Konturen durch eine Bilderfassung des Behandlungsobjekts bestimmt. Zusätzlich kann vorgesehen sein, daß es auch die Bestrahlungsflächen mit verschiedenen Bestrahlungsintensitäten auf der Grundlage der Erfassung von Konturen der verschiedenen Teilbereiche des Behandlungsobjekts bestimmt.

Da Bilderfassungen in der Regel vor der Vornahme einer Bestrahlung aus medizinischer Sicht bearbeitet werden, um einen Behandlungsplan zu erstellen, ist es zweckmäßig, wenn das Programm die Steuerung der Antriebseinrichtung auf der Grundlage einer Bearbeitung der Bilderfassung durch einen Behandlungsplan vornimmt. Auch hier kann eine Bestimmung von Teilfeldsegmenten vorgenommen werden.

Selbstverständlich sind bezüglich des Programm noch viele Modifikationen denkbar, hier sind nur die wesentlichen Arbeitsschritte aufgeführt, die ein Rechner vollziehen muß, um als Steuerung des Kollimators zu dienen.

Die Erfindung wird nachfolgend anhand der Zeichnung erläutert. Es zeigen
- **Fig.1**: eine Prinzipienskizze eines Kollimators, der durch die Erfindung weitergebildet wurde, wobei jedoch auch einige erfindungsgemäße Ausgestaltungen aufgenommen wurden,
- **Fig. 2**: ein Ausführungsbeispiel der Erfindung in perspektivischer Ansicht,
- **Fig. 3**: dasselbe Ausführungsbeispiel aus anderer Perspektive mit einem weiteren Abschirmungsblock,
- **Fig. 4**: einen Ausschnitt aus einer Ausgestaltung des erfindungsgemäßen Kollimators,
- **Fig. 5**: das Prinzip eines durch ein Programm realisierten Bestrahlungskonzepts,
- **Fig. 6**: eine weitere Ausgestaltung des Bestrahlungskonzepts mit Hinweis auf die durch die Erfindung ermöglichte Ausgestaltung und
- **Fig. 6a**: einen Ausschnitt aus Fig. 6.

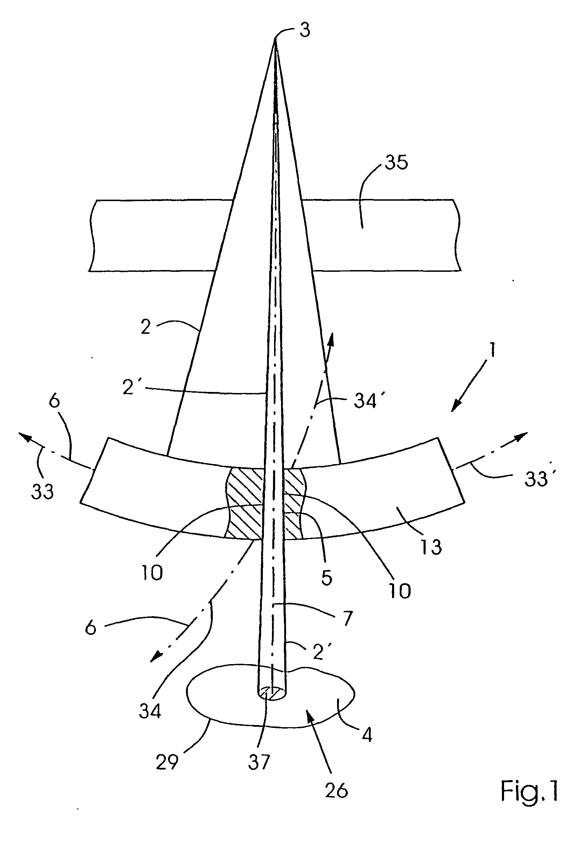

**Fig. 1** zeigt eine Prinzipskizze eines Kollimators, der durch die Erfindung weitergebildet wurde. Dieses Prinzip besteht darin, daß eine Kollimatoröffnung 5, die von einer Strahlungsquelle 3 kommenden Strahlen 2 derart begrenzt, daß eine Strahlenbeaufschlagung mit einer Fläche 37 entsteht, die wesentlich kleiner ist, als die Fläche 26 der vorzunehmenden Bestrahlung. Die Kollimatoröffnung 5 befindet sich in einem Abschirmungsblock 13, welcher auf einer Bahn 6 derart verschiebbar ist, daß die durch die Kollimatoröffnung 5 fallenden Strahlen 2' mittels eines entsprechenden Antriebs 8, der hier nicht eingezeichnet ist, die zu bestrahlende Fläche 26 abscannen und diese so mit der gewünschten Bestrahlung beaufschlagen. Die Bestrahlungsfläche 26 entspricht dabei der Form des Behandlungsobjekts 4 aus der Bestrahlungsrichtung 27 der aktuell vorgenommenen Bestrahlung. Dies wird weiter unten noch erläutert.

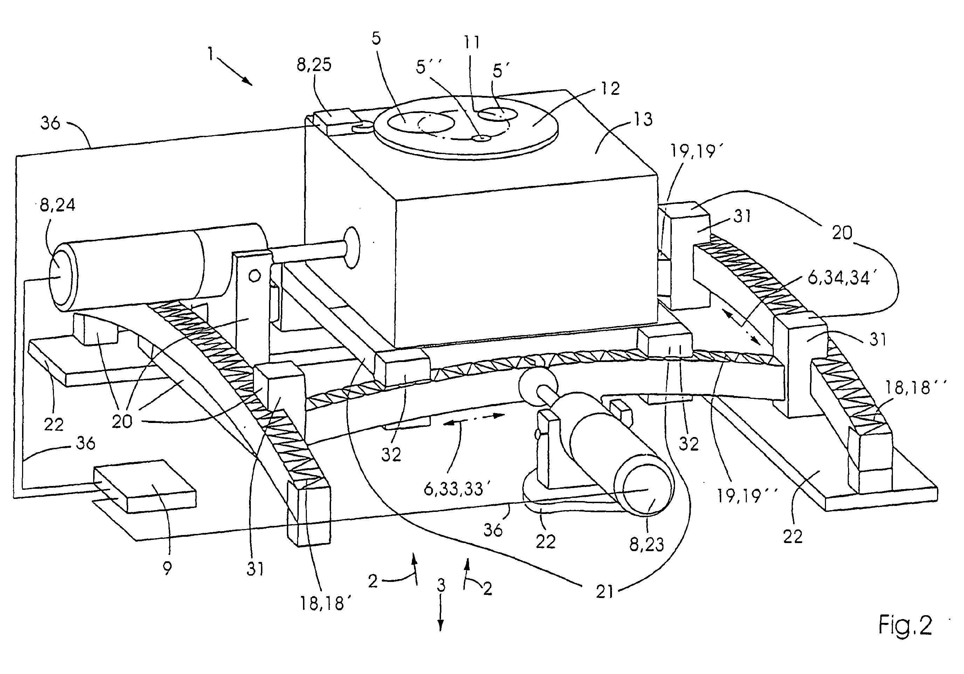

Einige erfindungsgemäße Ausgestaltungen wurden in die Zeichnung aufgenommen: Die Verschiebung des Abschirmungsblock 13 mit der Kollimatoröffnung 5 auf einer kugeloberflächenförmigen Bahn 6 findet dadurch statt, daß bezogen auf die Kugeloberfläche Scannbewegungen 33, 33' in der x-Richtung und Scannbewegungen 34, 34' in der y-Richtung vorgenommen werden. Dabei ist die Kollimatoröffnung 5 derart ausgerichtet, daß ihre Mittelachse 7 zur Strahlungsquelle 3 weist. Außerdem sind die Begrenzungen 10 der Kollimatoröffnung 5 konisch verlaufend in Richtung des Strahlengangs 2, 2' ausgerichtet, so daß zur Abschirmung immer die volle Stärke des Abschirmblocks 13 zur Verfügung steht und kein Halbschatten durch unzureichende Abschirmung entsteht. Der Abschirmungsblock 13 muß dann unter Aufrechterhaltung dieser Ausrichtung der Kollimatoröffnung 5 geführt werden. Ein Ausführungsbeispiel für eine derartige Führung zeigt Fig. 2, wobei selbstverständlich auch andere Arten von Führungen möglich sind.

Weiterhin zeigt die **Fig. 1,** daß die von der Strahlungsquelle 3 kommenden Strahlen 2' in üblicher Weise durch einen Vorkollimator 35 begrenzt werden, wobei die Öffnung dieses Vorkollimators 35 derart bemessen ist, daß er alle Bereiche abschirmt, die außerhalb der Abschirmung des Abschirmungsblocks 13 liegen, so daß der Abschirmungsblock 13 die durch den Vorkollimator hindurchtretenden Strahlen 2 in jeder seiner möglichen Positionen abschirmt, bis auf die Strahlen 2', die durch seine Kollimatoröffnung 5 hindurchtreten. Selbstverständlich könnte die Öffnung des Vorkollimators 35 auch variabel oder ebenfalls verschiebbar sein.

Damit die Kontur 29 der Begrenzung der Bestrahlungsfläche 26 möglichst exakt nachgefahren werden kann, werden im Kollimator 1 erfindungsgemäß auch kleinere Kollimatoröffnungen 5' oder 5" vorgesehen, was in den nachfolgenden Darstellungen in Form eines Ausführungsbeispiels der Erfindung gezeigt wird.

**Fig. 2** zeigt ein Ausführungsbeispiel der Erfindung in perspektivischer Ansicht, wobei bei dieser Darstellung sich die Strahlungsquelle 3 unterhalb des dargestellten Kollimators 1 befindet und die Strahlen 2 aus dieser Richtung auf den Kollimator 1 treffen. Strahlenquelle 3 und Vorkollimator 35 sind der Einfachheit halber weggelassen.

Bei diesem Ausführungsbeispiel ist dargestellt, wie ein Antrieb 8 realisiert werden kann. Um Bewegungen auf der kugeloberflächenförmigen Bahn 6 vornehmen zu können, sind zunächst eine erste Gleitschiene 18 in einem lediglich fragmentartig dargestellten Kollimatorgehäuse 22 angeordnet, wobei diese erste Gleitschiene 18 aus einem Schienenpaar 18' und 18" besteht, welches eine derart bogenförmige Form aufweist, daß die Mittelpunkte dieser beiden Bögen auf einer Achse liegen, welche durch die nahezu punktförmige Strahlungsquelle 3 hindurchgeht. Auf dieser ersten Gleitschiene 18 ist ein erster verschiebbarer Gleitschlitten 20 angeordnet, welcher auf der ersten Gleitschiene 18 laufende Lagerungen 31 und ein zweites Schienenpaar 19' und 19" aufweist, das eine zweite Gleitschiene 19 bildet, die senkrecht zur ersten Gleitschiene 18 verläuft. Auch das Schienenpaar 19' und 19" der zweiten Gleitschiene 19 sind bogenförmig ausgebildet, wobei auch hier die Mittelpunkte dieser Bögen auf einer Achse liegen, die durch die Strahlungsquelle 3 verläuft. Auf der zweiten Gleitschiene 19 ist ein zweiter Gleitschlitten 21 angeordnet, welcher mittels Lagerungen 32 verschiebbar ist. Auf diesem zweiten Gleitschlitten 21 befindet sich der Abschirmungsblock 13, der die Kollimatoröffnungen 5, 5', 5" trägt.

Durch die beiden Gleitschlitten 20 und 21 ist eine Verschiebung auf der kugeloberflächenförmigen Bahn 6 möglich, und es lassen sich Scannbewegungen 33, 33' in x-Richtung und Scannbewegungen 34, 34' in y-Richtung ausführen. Dazu dienen ein Antrieb 23 des ersten Gleitschlittens 20, welcher am Kollimatorgehäuse 22 angeordnet ist. Dieser Antrieb dient zur Ausführung der Scannbewegungen 34, 34'. Auf dem ersten Gleitschlitten 20 ist ein Antrieb 24 für den zweiten Gleitschlitten 21 angeordnet, welcher der Verschiebung in x-Richtung, also der Ausführung der Scannbewegungen 33 und 33' dient. Auch hier beziehen sich die x- und γ-Richtung nicht auf eine gerade Fläche, sondern auf die Kugeloberfläche der kugeloberflächenförmigen Bahn 6.

Um Scannbewegungen 33, 33', 34, 34' zu erzielen, die derart ausgeführt werden, daß eine Bestrahlungsfläche 26 mit der vorbestimmten Bestrahlung beaufschlagbar ist, ist eine Steuerung 9 angeordnet, welche durch Anschlüsse 36 mit den Antrieben 23 und 24 verbunden ist. Ein Ausführungsbeispiel für die Durchführung einer solchen Scannbewegung mittels der Steuerung 9 wird noch weiter unten erläutert.

Das dargestellte Ausführungsbeispiel des Kollimators 1 ist weiterhin mit einer Einrichtung zum automatischen Wechseln der Kollimatoröffnungen 5, 5', 5" ausgestattet. Zu diesem Zweck ist im Abschirmungsblock 13 ein zylindrisches Teil 12 gelagert, welches drehbar ist und mittels eines weiteren Antriebs 25 in vorbestimmte Positionen gebracht werden kann. Auch dieser weitere Antrieb 25 ist mittels eines Anschlusses 36 mit der Steuerung 9 verbunden, welche die Stellbefehle zum Wechseln der Kollimatoröffnungen 5, 5', 5" gibt. Die Anordnung der Kollimatoröffnungen 5, 5', 5" ist derart, daß sich immer eine dieser Kollimatoröffnungen 5, 5' oder 5" in der Arbeitsposition befindet, in der ihre Mittelachse 7 zur Strahlungsquelle 3 ausgerichtet ist, wie dies bereits in Fig. 1 erläutert wurde. Zweckmäßigerweise ist die Anordnung der Kollimatoröffnungen 5, 5', 5" derart gewählt, daß sie sich auf einer gedachten Kreisbahn 11 bewegen, welche derart bemessen ist, daß sich die Kollimatoröffnungen 5, 5', 5" durch eine Drehung des zylindrischen Teils 12 in die Arbeitsposition bewegen lassen.

Bei dieser Ausführungsform ist selbstverständlich erforderlich, daß die nicht in der Arbeitsposition befindlichen Kollimatoröffnungen 5, 5'; 5', 5" oder 5, 5" in einem abgeschirmten Bereich befinden. Dies könnte durch eine entsprechende Ausbildung des Vorkollimators 35 vorgenommen werden oder in der Art und Weise wie dies in **Fig. 3** dargestellt ist.

**Fig. 3** zeigt dasselbe Ausführungsbeispiel wie die Fig. 1 aus anderer Perspektive, aus der der weitere Abschirmungsblock 17 sichtbar ist, der die hier nicht in der Arbeitsposition befindlichen Kollimatoröffnungen 5 und 5' abschirmt. Nur die in ihrer Arbeitsposition befindliche Kollimatoröffnung 5" ist nicht abgeschirmt. Dieser weitere Abschirmungsblock 17 kann am zweiten Gleitschlitten 21, beispielsweise am ersten Abschirmungsblock 13 angebracht sein. Um weniger Gewicht bewegen zu müssen, ist es jedoch auch möglich, den weiteren Abschirmungsblock 17 am Kollimatorgehäuse 22 anzubringen, dann ist erforderlich, daß dessen Größe und die Stellbewegungen der Scannbewegung derart aufeinander abgestimmt sind, daß sich die nicht in der Arbeitsposition befindlichen Kollimatoröffnungen 5 und 5' immer im Bereich der Abschirmung des weiteren Abschirmungsblock 17 befinden. Selbstverständlich könnte auch dieser verschiebbar sein.

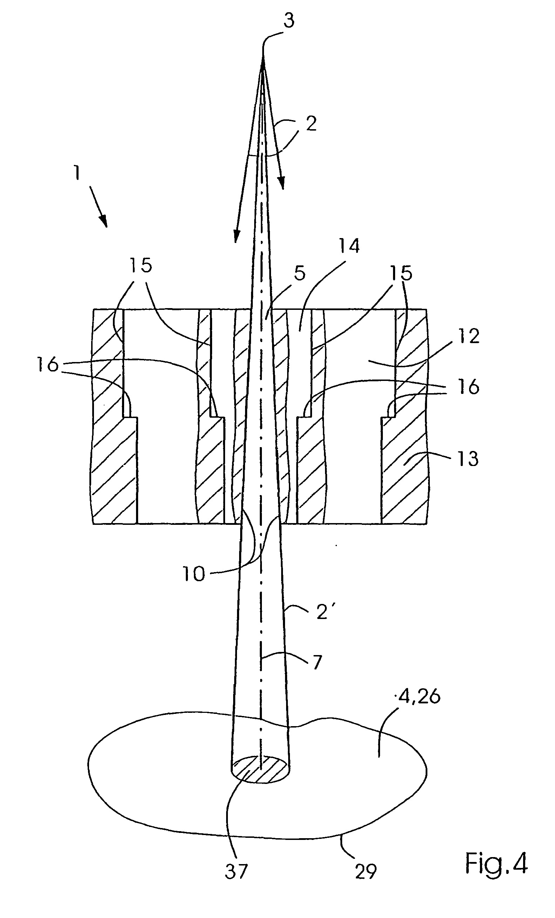

**Fig. 4** zeigt einen Ausschnitt aus einer Ausgestaltung eines erfindungsgemäßen Kollimators 1. Es kann sich dabei um den in den Fig. 2 und 3 dargestellten handeln oder auch um einen andersartig ausgestalteten erfindungsgemäßen Kollimator 1. Jedenfalls besteht dieser Kollimator 1 aus einem Abschirmungsblock 13, in dem ein zylindrisches Teil 12 drehbar gelagert ist, das Kollimatoröffnungen 5, 5' und 5" aufweist. Bei der Darstellung ist durch einen teilweisen Schnitt des Abschirmungsblock 13 die Ausgestaltung und Einfügung des zylindrischen Teils 12 sichtbar und es ist weiterhin sichtbar, daß die Kollimatoröffnung 5 auf einer Hülse 14 angeordnet ist, die in dem zylindrischen Teil 12 gelagert ist. Zweckmäßigerweise sind dann auch die anderen Kollimatoröffnungen 5' und 5" mittels Hülsen 14 in das zylindrische Teil 12 eingefügt, diese sind jedoch nicht eingezeichnet. Der Zweck dieser Ausgestaltung besteht darin, daß durch die Auswechselbarkeit der Kollimatoröffnungen 5, 5', 5" beliebige Größen dieser Kollimatoröffnungen 5, 5', 5" in den Kollimator 1 einfügbar sind, je nach dem, welche Bedürfnisse für die Bestrahlung bestehen, ob große Kollimatoröffnungen 5 für große Flächen 26 oder mittlere 5' beziehungsweise kleine Kollimatoröffnungen 5" für eine genaue Nacharbeitung von Konturen 29 der zu bestrahlenden Felder 26 erforderlich sind.

In der Darstellung ist weiterhin gezeigt, daß die Berührungsflächen 15 zweckmäßigerweise Absätze 16 aufweisen, damit auch dann keine Leckstrahlung durch sie hindurchgehen kann, wenn sie in einer bestimmten Stellung des Abschirmungsblocks 13 in einem Bereich in Richtung des Strahlengangs 2 verlaufen.

Selbstverständlich wäre auch eine Ausgestaltung möglich, bei der die zylindrischen Teile 12 gewechselt werden, um mehr als die auf dem Teil 12 befindlichen verschieden großen Kollimatoröffnungen 5, 5', 5" zur Verfügung zu haben. Die Lösung mit den Hülsen 14 hat jedoch den Vorteil, daß sie wegen des teueren Abschirmungsmaterials billiger ist und es hat den weiteren Vorteil, daß der Antrieb 25, der in Fig. 4 allerdings weggelassen wurde, für den Wechsel der Hülsen 14 nicht außer Eingriff gebracht werden muß, was bei einem Wechsel der zylindrischen Teile 12 der Fall wäre.

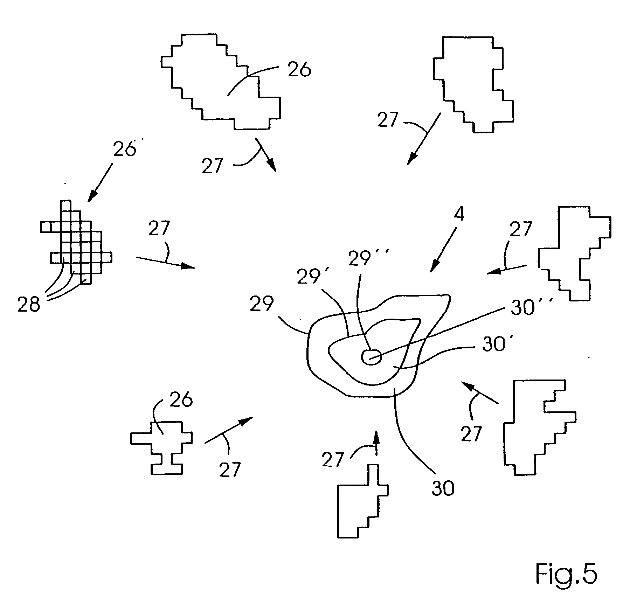

**Fig. 5** zeigt das Prinzip eines durch ein Programm realisierten Bestrahlungskonzepts. Die Bestrahlung von Behandlungsobjekten 4 wird in der Regel aus verschiedenen Bestrahlungsrichtungen 27 vorgenommen, um eine hohe Bestrahlung des Behandlungsobjekts 4 bei relativ geringerer Bestrahlung des umliegenden Gewebes zu erreichen. Da Behandlungsobjekte 4 eine unregelmäßige Gestalt aufweisen, muß für jede Bestrahlungsrichtung 27 eine Bestrahlungsfläche 26 gewählt werden, die der Form des Behandlungsobjekts 4 - aus der jeweiligen Bestrahlungsrichtung 27 betrachtet - entspricht. Zweckmäßigerweise wird die Form des Behandlungsobjekts 4 durch eine Bildgebung erfaßt, durch die zumindest die Bestrahlungsflächen 26 aus den verschiedenen Bestrahlungsrichtungen 27 ermittelt werden. Um die Bestrahlung dieser Flächen 26 durchführen zu können, ordnet ein Programm zweckmäßigerweise diesen Bestrahlungsflächen 26 Teilfeldsegmente 28 zu, um diese Teilfeldsegmente 28 bei der Durchführung der Bestrahlung mit den entsprechenden Strahlen 2' zu applizieren. Die Applikation quadratisch begrenzter Strahlen ist beispielhaft zur besseren Darstellung des Prinzips gezeigt. Zur Vermeidung von Stufen sind runde Kollimatoröffnungen 5, 5', 5" vorzuziehen.

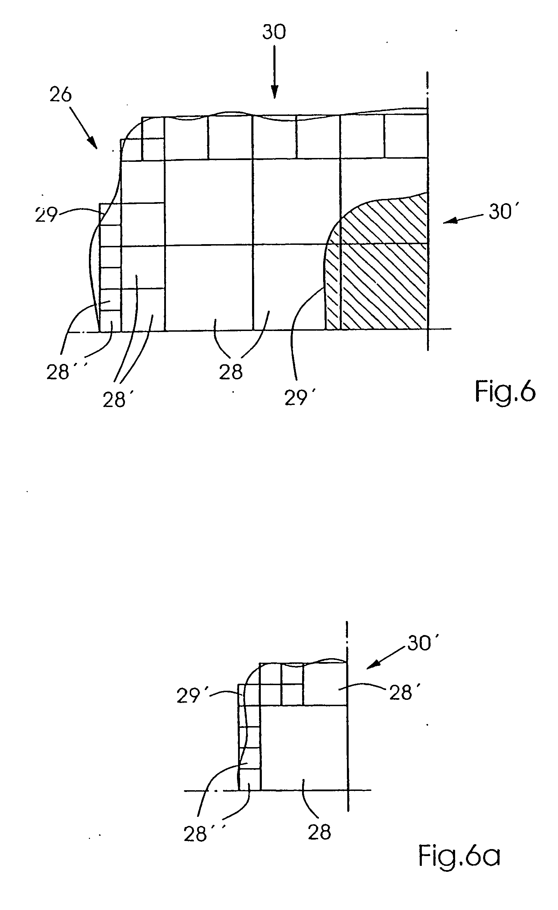

Das Behandlungsobjekt 4 weist in der Darstellung eine Kontur 29 auf, die nicht den eingezeichneten Bestrahlungsrichtungen 27 entspricht, sondern der Richtung des Betrachters. Tatsächlich muß man sich das Behandlungsobjekt 4 als räumliches Gebilde vorstellen. Entsprechend anders geformte Konturen 29, 29', 29" sowohl für das Behandlungsobjekt 4 als auch für Bereiche desselben, die mit einer höheren Intensität 30', 30" bestrahlt werden müssen, ergeben sich für jede Bestrahlungsrichtung 27. An den dargestellten Konturen 29, 29', 29" ist jedoch das Prinzip erkennbar, das darauf beruht, daß das Bestrahlungsobjekt 4 nicht gleichmäßig verteilt mit derselben Intensität bestrahlt werden soll, sondern daß verschiedene Intensitäten 30, 30', 30" vorgesehen werden, die jeweils durch Konturen 29, 29', 29" begrenzt sind. Um dies zu erreichen, ist eine weitere Ausgestaltung des Bestrahlungskonzepts zweckmäßig, die anhand der **Fig. 6 und 6a** erläutert wird.

**Fig. 6** zeigt eine solche weitere Ausgestaltung des Bestrahlungskonzepts, wobei beispielhaft zwei Bestrahlungsintensitäten 30 und 30' dargestellt sind. Dabei ist nicht die gesamte Bestrahlungsfläche 26 dargestellt, sondern lediglich ein Ausschnitt, der durch die strichpunktierten Linien begrenzt ist. Sichtbar sind die beiden Konturen 29 und 29' für die vorzusehenden Bestrahlungsintensitäten 30, 30'. Die verschiedenen Bestrahlungsintensitäten 30 und 30' können dadurch erzielt werden, daß zuerst die in Fig. 6 dargestellte Fläche 26 mit einer bestimmten Bestrahlungsintensität 30 beaufschlagt wird und dann eine nachfolgende nochmalige Bestrahlung innerhalb der Kontur 29' stattfindet, um die Intensität 30' zu erzielen. Selbstverständlich wäre es jedoch auch möglich, den Bereich zwischen den Konturen 29 und 29' zuerst mit einer ersten Intensität 30 zu beaufschlagen und danach den Bereich, der von der Kontur 29' eingegrenzt wird mit einer zweiten Intensität 30'.

**Fig. 6** und **Fig. 6a** stellen jedoch gegenüber der Darstellung in Fig. 5 noch eine weitere Ausgestaltung der Bestrahlung dar, bei der erfindungsgemäß Kollimatoröffnungen 5, 5', 5" verschiedener Größen zur Verfügung stehen. Auf diese Weise können die Bestrahlungsflächen 26 in Teilfeldsegmente 28, 28', 28" verschiedner Größen unterteilt werden, damit es möglich ist, verschiedene Konturen 29 sowie verschiedene Konturen 29, 29', 29" verschieden hoher Bestrahlungsintensitäten 30, 30', 30" nachvollziehen zu können. Die Darstellung ist vereinfacht an zwei Bestrahlungsintensitäten 30 und 30' dargestellt, wobei die **Fig. 6** eine erste Bestrahlung in großen Teilfeldsegmenten 28 und je nach Verlauf der Kontur 29 mit mittleren Teilfeldsegmenten 28' und kleinen Teilfeldsegmenten 28" zeigt. Danach erfolgt gemäß **Fig. 6a** eine nochmalige Bestrahlung des Bereichs, der mit der höheren Intensität 30' beaufschlagt werden muß. Auch hier wird die Kontur 29' mittels der Teilfeldsegmente 28, 28' und 28" ausgefüllt. Inwieweit an der Kontur selbst im Zweifel umliegendes Gewebe geringfügig mitbestrahlt werden soll oder Teile innerhalb der Kontur 29 oder 29' nicht erfaßt werden sollen, ist dabei eine ärztliche Entscheidung. Statt einer zweimaligen Bestrahlung kann auch die Scanngeschwindigkeit unterschiedlich sein, um verschiedene Bestrahlungsintensitäten 30, 30', 30" zu erzielen. Auch dabei kann das Feld zuerst mit großen Kollimatoröffnungen 5, 5' grob und danach mit kleinen Kollimatoröffnungen 5', 5" mit exakten Begrenzungen abgescannt werden.

Selbstverständlich sind jedoch auch andere Ausgestaltungen der Erfindung möglich. Beispielsweise könnte ein Abschirmungsblock 13 kugelschalenähnlich geformt sein, also ähnlich wie die Darstellung in **Fig. 1,** wobei eine solche Kugelschale auf einem Ring lagert, welcher zu dieser Form komplementär ist. Auch dann wäre eine Verschiebung auf einer kugeloberflächenförmigen Bahn 6 möglich. Es wäre auch denkbar, in einem solchen Abschirmungsblock 13 lediglich eine Kollimatoröffnung 5 einzufügen, wobei diese in einer Hülse 14 angeordnet sein kann, um die Größenveränderung der Kollimatoröffnung 5, 5', 5", .., durch den Wechsel der Hülsen 14 vorzunehmen. Oder es ist auch möglich, die Kollimatoröffnung 5, 5', 5" und vielleicht weiterer Kollimatoröffnungen in anderer Weise anzuordnen, die jeweils gewünschte in die Arbeitsposition zu verbringen und die anderen abzuschirmen.

Bezüglich der Ausgestaltung des Bestrahlungskonzepts sind selbstverständlich auch andere Vorgehensweisen denkbar, beispielsweise, daß die Konturen 29, 29' oder 29" mittels der Flächen 37 der Strahlenbeaufschlagung in analoger Weise direkt nachgezogen werden und daß dann der verbleibende Rest der Flächen 26, gegebenenfalls mit größeren Kollimatoröffnungen ausgefüllt wird. Dazu sind insbesondere runde Kollimatoröffnungen 5, 5', 5" geeignet. Eine derartig Ausfüllung kann auch so vorgenommen werden, daß nach der Kontur 29, 29' oder 29" immer der innere Rand der bereits bestrahlten Fläche nachgefahren wird, bis man die Fläche 26 völlig ausgefüllt hat. Weitere Ausgestaltungen des Kollimators 1 sowie des Bestrahlungskonzepts, das der Steuerung 9 beziehungsweise dem Programm zugrundeliegt, sind denkbar.

### Kollimator und Programm zur Steuerung des Kollimators

### Bezugszeichenliste

- 1: Kollimator
- 2,2': Strahlen / Strahlengang
- 2': Strahlen durch den Kollimator 1 begrenzt
- 3: Strahlungsquelle
- 4: Behandlungsobjekt
- 5, 5', 5": Kollimatoröffnungen
- 5: groß
- 5': mittel
- 5": klein
- 6: (kugeloberflächenförmige) Bahn
- 7: Mittelachse der Kollimatoröffnung
- 8: Antriebseinrichtung
- 9: Steuerung
- 10: Begrenzungen der Kollimatoröffnung
- 11: gedachte Kreisbahn
- 12: zylindrisches Teil
- 13: Abschirmungsblock
- 14: Hülsen
- 15: Berührungsflächen
- 16: Absätze
- 17: weiterer Abschirmungsblock
- 18: erste Gleitschiene
- 18', 18": erstes Schienenpaar
- 19: zweite Gleitschiene
- 19', 19": zweites Schienenpaar
- 20: erster Gleitschlitten
- 21: zweiter Gleitschlitten
- 22: Kollimatorgehäuse (fragmentartig dargestellt)
- 23: Antrieb des ersten Gleitschlittens
- 24: Antrieb des zweiten Gleitschlittens
- 25: weiterer Antrieb
- 26: Bestrahlungsfläche / Fläche der vorzunehmenden Bestrahlung
- 27: Bestrahlungsrichtungen
- 28, 28', 28": Teilfeldsegmente
- 29, 29', 29": Kontur
- 29', 29": Kontur einer Bestrahlungsfläche höherer Intensität
- 30, 30', 30": Bestrahlungsintensitäten
- 30', 30": höhere Intensitäten
- 31: Lagerungen des ersten Gleitschlittens
- 32: Lagerungen des zweiten Gleitschlittens
- 33, 33': Scannbewegung in x-Richtung
- 34, 34': Scannbewegung in y-Richtung
- 35: Vorkollimator
- 36: Anschlüsse der Steuerung an die Antriebe
- 37: Fläche der Strahlenbeaufschlagung durch den vom Kollimator begrenzten Strahl 2'

## Patentansprüche

1. Kollimator (1) zum Begrenzen eines Bündels energiereicher Strahlen (2), das von einer im wesentlichen punktförmigen Strahlungsquelle (3) ausgehend auf ein Behandlungsobjekt (4) gerichtet ist und insbesondere der stereotaktischen Konformationsbestrahlung von Tumoren dient, wobei der Kollimator (1) derart ausgebildet ist, daß ein unregelmäßiges Behandlungsobjekt (4) mittels Strahlen (2') abscannbar ist, die durch eine Kollimatoröffnung begrenzt sind,
**dadurch gekennzeichnet,**
**daß** er mehrere Kollimatoröffnungen (5, 5', 5") in verschiedenen Größen aufweist, wobei wahlweise eine (5, 5', 5") davon auf einer kugeloberflächenförmigen Bahn (6) allseitig verschiebbar und mit ihrer Mittelachse (7) auf die Strahlungsquelle (3) ausgerichtet ist und wobei die anderen Kollimatoröffnungen (5', 5") von den Strahlen (2) abgeschirmt sind, und
**daß** er eine Steuerung (9) aufweist, die so ausgebildet ist, daß sie auf die Antriebe (23, 24, 25) einer Antriebseinrichtung (8) derart wirkt, daß für das Abscannen großer Bestrahlungsflächen (26) des Behandlungsobjekts (4) große Kollimatoröffnungen (5, 5') und für die exakte Begrenzung am Rande der Bestrahlungsflächen (26) des Behandlungsobjekts (4), insbesondere bei unregelmäßigen Konturen (29, 29', 29"), kleine Kollimatoröffnungen (5', 5") eingesetzt werden.

2. Kollimator nach Anspruch 1,
**dadurch gekennzeichnet,**
**daß** die mindestens eine Kollimatoröffnung (5, 5', 5") in Richtung des Strahlengangs (2, 2') verlaufende Begrenzungen (10) aufweist.

3. Kollimator nach Anspruch 1 oder 2,
**dadurch gekennzeichnet,**
**daß** die mindestens eine Kollimatoröffnung (5, 5', 5") rund ist.

4. Kollimator nach Anspruch 1 oder 2,
**dadurch gekennzeichnet,**
**daß** die mindestens eine Kollimatoröffnung (5, 5', 5") viereckig ist.

5. Kollimator nach einem der Ansprüche 1 bis 4,
**dadurch gekennzeichnet,**
**daß** mehrere Kollimatoröffnungen (5,5',5") um eine gedachte Kreisbahn (11) drehbar angeordnet sind.

6. Kollimator nach Anspruch 5,
**dadurch gekennzeichnet,**
**daß** sich die Kollimatoröffnungen (5, 5', 5") auf einem zylindrischen Teil (12) aus abschirmendem Material befinden, das durch einen revolverähnlichen Mechanismus drehbar und mindestens in den Arbeitspositionen festlegbar ist.

7. Kollimator nach Anspruch 6,
**dadurch gekennzeichnet,**
**daß** das zylindrische Teil (12) in einem Abschirmungsblock (13) drehbar gelagert ist.

8. Kollimator nach Anspruch 1 bis 7,
**dadurch gekennzeichnet,**
**daß** die mindestens eine Kollimatoröffnung (5, 5', 5") gegen mindestens eine Kollimatoröffnung einer anderen Größe austauschbar ist.

9. Kollimator nach Anspruch 8,
**dadurch gekennzeichnet,**
**daß** ein Satz von Kollimatoröffnungen (5, 5', 5",...) verschiedener Größen mittels austauschbarer Hülsen (14) aus abschirmendem Material in den Kollimator (1) einfügbar sind.

10. Kollimator nach Anspruch 6 bis 9,
**dadurch gekennzeichnet,**
**daß** die Aneinanderfügungen abschirmender Bauteile (12, 13, 14) keine parallel zum Strahlengang (2) verlaufende durchgehende Berührungsflächen (15) aufweisen.

11. Kollimator nach Anspruch 6 bis 10,
**dadurch gekennzeichnet,**
**daß** Berührungsflächen (15) der Bauteile (12, 13, 14) Absätze (16) aufweisen.

12. Kollimator nach Anspruch 1 bis 11,
**dadurch gekennzeichnet,**
**daß** der Abschirmung der nicht in der Arbeitsposition befindlichen Kollimatoröffnungen (5, 5'; 5', 5" oder 5, 5") ein weiterer Abschirmungsblock (17) dient.

13. Kollimator nach Anspruch 12,
**dadurch gekennzeichnet,**
**daß** der weitere Abschirmungsblock (17) am Abschirmungsblock (13) angeordnet ist.

14. Kollimator nach Anspruch 13,
**dadurch gekennzeichnet,**
**daß** der weitere Abschirmungsblock (17) feststehend am Kollimator (1) angeordnet ist und seine Ausdehnung derart bemessen ist, daß die sich nicht in der Arbeitsposition befindlichen Kollimatoröffnungen (5, 5'; 5', 5" oder 5, 5") bei jeder Verschiebung der in der Arbeitsposition liegenden Kollimatoröffnung (5", 5 oder 5') gegenüber den Strahlen (2) im Schatten des weiteren Abschirmungsblocks (17) liegen.

15. Kollimator nach einem der Ansprüche 1 bis 14,
**dadurch gekennzeichnet,**
**daß** die kugeloberflächenförmige Bahn (6) durch zwei senkrecht zueinander verlaufende Gleitschienen (18, 19) gebildet wird, auf denen der Abschirmblock (13) verschiebbar ist.

16. Kollimator nach Anspruch 15,
**dadurch gekennzeichnet,**
**daß** die Gleitschienen (18, 19) durch Schienenpaare (18', 18"; 19', 19") gebildet sind, wobei ein erstes Schienenpaar (18', 18") fest mit dem am Bestrahlungsgerät anordenbaren Kollimatorgehäuse (22) verbunden ist, das zweite Schienenpaar (19', 19") einem ersten Gleitschlitten (20) zugeordnet ist, der auf dem ersten Schienenpaar (18', 18") läuft und daß der Abschirmblock (13) auf einem zweiten Gleitschlitten (21) angeordnet ist, der auf dem zweiten Schienenpaar (19', 19") läuft.

17. Kollimator nach einem der Ansprüche 1 bis 16,
**dadurch gekennzeichnet,**
**daß** die Antriebseinrichtung (8) zur Verschiebung der mindestens einen Kollimatoröffnung (5, 5', 5") auf der kugeloberflächenförmigen Bahn (6) zwei Antriebe (23, 24) aufweist.

18. Kollimator nach Anspruch 16 und 17,
**dadurch gekennzeichnet,**
**daß** die Antriebe (23, 24) zur Bewegung der Gleitschlitten (20, 21) an diesen angeordnet sind.

19. Kollimator nach Anspruch 17 oder 18,
**dadurch gekennzeichnet,**
**daß** die Antriebe (23, 24) mit Kugelgewindespindeln ausgestattet sind.

20. Kollimator nach Anspruch 17 oder 18,
**dadurch gekennzeichnet,**
**daß** die Antriebe (23, 24) mit Rollengewindespindeln ausgestattet sind.

21. Kollimator nach einem der Ansprüche 1 bis 20,
**dadurch gekennzeichnet,**
**daß** die Antriebseinrichtungen (8) einen weiteren Antrieb (25) zur Verbringung der gewünschten Kollimatoröffnung (5, 5' oder 5") in die Arbeitsposition aufweist.

22. Kollimator nach Anspruch 21,
**dadurch gekennzeichnet,**
**daß** der weitere Antrieb (25) auf dem Abschirmungsblock (13) angeordnet ist, um das zylindrische Teil (12) in die Arbeitspositionen der Kollimatoröffnungen (5, 5' oder 5") zu drehen.

23. Kollimator nach einem der Ansprüche 1 bis 22,
**dadurch gekennzeichnet,**
**daß** die Antriebseinrichtung (8) mindestens einen elektromotorischen Antrieb (23, 24, 25) aufweist.

24. Kollimator nach einem der Ansprüche 1 bis 23,
**dadurch gekennzeichnet,**
**daß** die Antriebseinrichtung (8) mindestens einen pneumatischen Antrieb (23, 24, 25) aufweist.

25. Kollimator nach einem der Ansprüche 1 bis 24,
**dadurch gekennzeichnet,**
**daß** die Antriebseinrichtung (8) mindestens einen hydraulischen Antrieb (23, 24, 25) aufweist.

26. Kollimator nach einem der Ansprüche 1 bis 25,
**dadurch gekennzeichnet,**
**daß** die Steuerung (9) derart ausgebildet ist, daß die Verweilzeit der Strahlen (2) mittels der Antriebseinrichtung (8, 23, 24) steuerbar ist.

27. Kollimator nach Anspruch 26,
**dadurch gekennzeichnet,**
**daß** die Steuerung (9) derart ausgebildet ist, daß die Verweilzeit durch die Scanngeschwindigkeit der Strahlen (2') erzielbar ist, mit der diese das Behandlungsobjekt (4) überstreichen.

28. Kollimator nach einem der Ansprüche 1 bis 27,
**dadurch gekennzeichnet,**
**daß** die Steuerung (9) ein mittels eines entsprechenden Programms geladener Rechner ist.

29. Kollimator nach einem der Ansprüche 1 bis 28,
**dadurch gekennzeichnet,**
**daß** er mit Positionserfassungseinrichtungen für die Stellbewegungen ausgestattet ist.

30. Kollimator nach einem der Ansprüche 1 bis 29,
**dadurch gekennzeichnet,**
**daß** die Steuerung (9) derart ausgebildet ist, daß der Scannvorgang mittels eines vorgegebenen Behandlungsplans erfolgt.

31. Kollimator nach Anspruch 29 und 30,
**dadurch gekennzeichnet,**
**daß** die Steuerung (9) mit den Positionserfassungseinrichtungen verbunden und derart ausgestaltet ist, daß die Positionserfassungen der Verifikation und Korrektur der Stellbewegungen dienen.

32. Programm zur Steuerung eines Kollimators nach einem der Ansprüche 1 bis 31,
**dadurch gekennzeichnet,**
**daß** es derart ausgebildet ist, daß es mittels eines Rechners die Antriebseinrichtung (8, 23, 24) derart steuert, daß ein Behandlungsobjekt (4) entsprechend der vorbestimmten Konturen (29, 29', 29") bestrahlbar ist, wobei es durch Betätigung des weiteren Antriebs (25) die für die Ausbildung der Kontur (29, 29' , 29") optimale Größe einer Kollimatoröffnung (5, 5' oder 5") auswählt und die Scannvorgänge, die mit den verschiedenen Kollimatoröffnungen (5, 5', 5") ausgeführt werden, zur Erzielung der geforderten Bestrahlung aufeinander abstimmt.

33. Programm nach Anspruch 32,
**dadurch gekennzeichnet,**
**daß** es derart ausgebildet, ist, daß es die Bestrahlungsintensität (30, 30', 30") mittels der Verweilzeit durch eine entsprechende Steuerung der Antriebseinrichtung (8, 23, 24) variieren kann.

34. Programm nach Anspruch 32 oder 33,
**dadurch gekennzeichnet,**
**daß** es derart ausgebildet ist, daß es die jeweilige Bestrahlungsfläche (26) für ein Behandlungsobjekt (4) in Teilfeldsegmente (28, 28', 28") unterteilt und nach diesen Teilfeldsegmenten (28, 28', 28") die Bestrahlung mittels des Rechners steuert.

35. Programm nach Anspruch 34,
**dadurch gekennzeichnet,**
**daß** es die Unterteilung in Teilfeldsegmente (28, 28', 28") für die Bestrahlungsflächen (26) für verschiedene Bestrahlugsrichtungen (27), aus denen das Behandlungsobjekt (4) bestrahlt werden soll, vornimmt.

36. Programm nach einem der Ansprüche 32 bis 35,
**dadurch gekennzeichnet,**
**daß** es derart ausgebildet ist, daß es die Bestimmung der Bestrahlungsflächen (26) auf der Grundlage der Erfassung der Konturen (29, 29', 29") durch eine Bilderfassung des Behandlungsobjekts (4) bestimmt.

37. Programm nach Anspruch 36,
**dadurch gekennzeichnet,**
**daß** es auch die Bestrahlungsflächen (26) mit verschiedenen Bestrahlungsintensitäten (30, 30', 30") auf der Grundlage der Erfassung von Konturen (29, 29', 29") verschiedener Teilbereiche des Behandlungsobjekts (4) bestimmt.

38. Programm nach einem der Ansprüche 32 bis 37,
**dadurch gekennzeichnet,**
**daß** es derart ausgebildet ist, daß es die Steuerung der Antriebseinrichtung (8, 23, 24) auf der Grundlage einer Bearbeitung der Bilderfassung durch einen Behandlungsplan vornimmt.

## Claims

1. A collimator for defining a beam of high energy rays (2) which is emitted from a substantially punctiform radiation source (3) and directed onto an object for therapy and serving to effect stereotactic conformation irradiation of tumors, the collimator being designed to scan an irregular object for therapy using rays (2') delimited by a collimator opening, **characterized in that** said collimator comprises a plurality of collimator openings (5, 5', 5") of various sizes, one (5, 5', 5") of which can be displaced in all directions over a spherically shaped path (6) with its center axis (7) being oriented toward the radiation source (3) while the other collimator openings (5', 5") are shielded from radiation (2), and that said collimator has control means (9) adapted to control the drive mechanisms (23, 24, 25) of drive means (8) such that large collimator openings (5', 5") are used for scanning large irradiation areas (26) of the object (4) for therapy and small collimator openings (5', 5") are used for precise delimitation at the edges of said irradiation areas, particularly in the case of irradiation areas having irregular contours (29, 29', 29").

2. A collimator as defined in Claim 1, **characterized in that** at least one collimator opening (5, 5', 5") has delimitations extending in the direction of the path of rays (2, 2').

3. A collimator as defined in Claim 1 or Claim 2, **characterized in that** at least one collimator opening (5, 5', 5") is round.

4. A collimator as defined in Claim 1 or Claim 2, **characterized in that** at least one collimator opening (5, 5', 5") is tetragonal.

5. A collimator as defined in any one of Claims 1 to 4, **characterized in that** a plurality of collimator openings (5, 5', 5") are rotatable about an imaginary circular path (11).

6. A collimator as defined in Claim 5, **characterized in that** said collimator openings (5, 5', 5") are located on a cylindrical element (12) of shielding material which can be rotated by a revolver-like mechanism and can be locked in at least its working position.

7. A collimator as defined in Claim 6, **characterized in that** said cylindrical element (12) is rotatably mounted in a shielding block (13).

8. A collimator as defined in any one of Claims 1 to 7, **characterized in that** at least one collimator opening (5, 5', 5") can be replaced by a collimator opening of a different size.

9. A collimator as defined in Claim 8, **characterized in that** a set of collimator openings (5, 5', 5", ...) of various sizes can be inserted into said collimator by means of replaceable sleeves (14) of shielding material.

10. A collimator as defined in any one of Claims 6 to 9, **characterized in that** adjacent shielding elements (12, 13, 14) have no continuous contact surfaces (15) extending parallel to the path of rays (2).

11. A collimator as defined in any one of Claims 6 to 10, **characterized in that** contact surfaces (15) of said elements (12, 13, 14) are provided with steps (16).

12. A collimator as defined in any one of Claims 1 to 11, **characterized in that** an additional shielding block (17) is provided to shield collimator openings (5, 5'; 5', 5"; or 5, 5") which are not in their working position.

13. A collimator as defined in Claim 12, **characterized in that** said additional shielding block (17) is disposed on said first shielding block (13).

14. A collimator as defined in Claim 13, **characterized in that** said additional shielding block (17) is fixed to said collimator and is dimensioned such that the collimator openings (5, 5'; 5', 5"; or 5, 5") not in a working position are shaded from the radiation (2) by said additional shielding block (17) whenever there is displacement of the collimator opening (5", 5; or 5') in its working position.

15. A collimator as defined in any one of Claims 1 to 14, **characterized in that** said spherically shaped path (6) is formed by slide rails (18, 19) disposed at right angles to each other and on which said shielding block (13) is mounted for displacement thereon.

16. A collimator as defined in Claim 15, **characterized in that** said slide rails (18, 19) are formed by rail pairs (18', 18"; 19', 19"), a first rail pair (18', 18") being securely attached to the collimator housing (22) capable of being mounted on the device for radiation therapy while the second rail pair (19'. 19") cooperates with a first carriage (20) which runs on said first rail pair (18', 18"), and said shielding block (13) is disposed on a second carriage (21) which runs on said second rail pair (19', 19").

17. A collimator as defined in any one of Claims 1 to 16, **characterized in that** said drive means (8) comprises two drive mechanisms (23, 24) to displace the at least one collimator opening (5, 5', 5") over said spherically shaped path (6).

18. A collimator as defined in Claim 16 and laim 17, **characterized in that** said drive mechanisms (23, 24) for driving said carriages (20, 21) are mounted on said carriages.

19. A collimator as defined in Claim 17 or Claim 18, **characterized in that** said drive mechanisms (23, 24) are equipped with ball screws.

20. A collimator as defined in any one of Claims 17 to 18, **characterized in that** said drive mechanisms (23, 24) are equipped with horizontal threaded spindles.

21. A collimator as defined in any one of Claims 1 to 20, **characterized in that** said drive means (8) comprise an additional drive mechanism (25) for moving the desired collimator opening (5, 5', 5") into its working position.

22. A collimator as defined in Claim 21, **characterized in that** said additional drive mechanism (25) is disposed on said shielding block (13) for rotation of the cylindrical element (12) into the working positions of said collimator openings (5, 5', 5").

23. A collimator as defined in any one of Claims 1 to 22, **characterized in that** said drive means (8) has at least one electromotive drive mechanism 23, 24, 25).

24. A collimator as defined in any one of Claims 1 to 23, **characterized in that** said drive means (8) comprises at least one pneumatic drive mechanism (23, 24, 25)).

25. A collimator as defined in any one of Claims 1 to 24, **characterized in that** said drive means (8) comprises at least one hydraulic drive mechanism (23, 24, 25).

26. A collimator as defined in any one of Claims 1 to 25, **characterized in that** said control means (9) is adapted to control the duration of irradiation (2) by means of said drive mechanisms (8, 23, 24).

27. A collimator as defined in Claim 26, **characterized in that** said control means (9) is adapted such that the duration of irradiation is defined by the scanning speed of the rays (2') sweeping over the object (4) for therapy.

28. A collimator as defined in any one of Claims 1 to 27, **characterized in that** said control means (9) consists of a computer running an appropriate program.

29. A collimator as defined in any one of Claims 1 to 28, **characterized in that** it is equipped with position detection means for effecting controlled movements.

30. A collimator as defined in any one of Claims 1 to 29, **characterized in that** said control means (9) is adapted such that the scanning operation is carried out in accordance with a predefined therapy schedule.

31. A collimator as defined in Claim 29 and Claim 30, **characterized in that** said control means (9) communicates with said position detection means such that said position detection means verifies and corrects the controlled movements.

32. A program for controlling a collimator as defined in any one of Claims 1 to 31, **characterized in that** it is adapted to control said drive mechanisms (8, 23, 24) implementing a computer such that an object (4) for therapy can be irradiated within predefined contours (29, 29', 29") and to select, by actuation of said further drive mechanism (25), an optimum size of a collimator opening (5, 5', or 5") for forming said contours (29, 29', 29"), and to adapt the scanning operations carried out with the various collimator openings (5, 5', 5") relatively to each other so as to achieve the required degree of irradiation.

33. A program as defined in Claim 32, **characterized in that** it is adapted to vary the intensity of irradiation (30, 30', 30") in terms of the duration thereof by appropriately controlling said drive mechanisms (8, 23, 24).

34. A program as defined in Claim 32 or Claim 33, **characterized in that** it is adapted to subdivide an area (26) to be irradiated on an object for therapy into subareas (28, 28', 28") and to control irradiation via said computer according to these subareas (28, 28', 28").

35. A program as defined in Claim 34, **characterized in that** the subdivision into subareas (28, 28', 28") of the area (26) to be irradiated is effected for the various directions (27) from which the object (4) for therapy will be irradiated.

36. A program as defined in any one of Claims 32 to 35, **characterized in that** it is adapted to determine the areas (26) to be irradiated on a basis of said contours (29, 29', 29") detected by imaging the object (4) for therapy.

37. A program as defined in Claim 36, **characterized in that** it determines the various areas (26) to be irradiated at different radiation intensities (30, 30', 30") on the basis of detected contours (29, 29', 29") of different subregions of the object (4) for therapy.

38. A program as defined in any one of Claims 32 to 37, **characterized in that** it effects control of said driving mechanisms (8, 23, 24) on the basis of image processing according to a therapy schedule.

## Revendications

1. Collimateur (1) pour la limitation d'un faisceau de rayons riches en énergie (2) qui est orienté depuis une source de rayonnement (3) sensiblement punctiforme sur un objet à traiter (4) et sert en particulier à l'irradiation de conformation de tumeurs stéréotactiques, le collimateur (1) étant réalisé de sorte qu'un objet de traitement irrégulier (4) peut être balayé au moyens de rayons (2') qui sont limités par une ouverture de collimateurs,
**caractérisé en ce**
**qu'**il présente plusieurs ouvertures de collimateurs (5, 5', 5'') de différentes dimensions, une ouverture (5, 5', 5") étant coulissante de part et d'autre sur une trajectoire de surface sphérique (6) et étant orientée par leur axe médian (7) sur la source de rayon (3) et les autres ouvertures de collimateur (5', 5") étant protégées des rayons (2) et en ce qu'il présente une commande (9) qui est réalisée de sorte qu'elle agit sur les entraînements (23, 24; 25) d'un dispositif d'entraînement (8) de sorte que pour le balayage de grandes surfaces d'irradiation (26) de l'objet à traiter (4), il est utilisé de grandes ouvertures de collimateurs (5, 5') et, pour la délimitation exacte au bord des surfaces d'irradiation (26) de l'objet à traiter (4), de petites ouvertures de collimateur (5', 5").

2. Collimateur selon la revendication 1, **caractérisé en ce qu'**au moins l'ouverture de collimateur (5, 5' 5") présente des délimitations (10) s'étendant en direction de la marche du rayon (2, 2').

3. Collimateur selon la revendication 1 ou 2, **caractérisé en ce qu'**au moins une ouverture de collimateur (5, 5', 5") est ronde.

4. Collimateur selon la revendication 1 ou 2, en ce qu'au moins une ouverture de collimateur (5, 5', 5") est quadrangulaire.

5. Collimateur selon l'une des revendications 1 à 4, **caractérisé en ce que** plusieurs ouvertures de collimateur (5. 5', 5'') sont disposées rotatives autour d'une orbite imaginaire (11).

6. Collimateur selon la revendication 5, **caractérisé en ce que** les ouvertures de collimateur (5, 5', 5'') se trouvent sur une partie cylindrique (12) en matériau de blindage qui est rotative par un mécanisme du type révolver et fixable au moins dans les positions de travail.

7. Collimateur selon la revendication 6, **caractérisé en ce que** la partie cylindrique (12) est logée rotative dans un bloc de blindage (13).

8. Collimateur selon les revendications 1 à 7, **caractérisé en ce que** au moins une ouverture de collimateur (5, 5', 5'') est échangeable contre au moins une ouverture de collimateur d'une autre dimension.

9. Collimateur selon la revendication 8, **caractérisé en ce qu'**un jeu d'ouvertures de collimateur (5, 5', 5", ...) de différentes dimensions sont insérables dans le collimateur (1) au moyen de gaines interchangeables (14) en matériau de blindage.

10. Collimateur selon la revendication 6 à 9, **caractérisé en ce que** l'assemblage de composants de blindage (12, 13, 14) ne présente aucune surface de contact (15) continue s'étendant parallèlement à la marche du rayon (2).

11. Collimateur selon la revendication 6 à 10, **caractérisé en ce que** des surfaces de contact (15) des composants (12, 13, 14) présentent des talons (16).

12. Collimateur selon la revendication 1 à 11, **caractérisé en ce que** le blindage des orifices de collimateur (5, 5'; 5', 5" ou 5, 5") ne se trouve pas dans la position de travail.

13. Collimateur selon la revendication 12, **caractérisé en ce que** l'autre bloc de blindage (17) est disposé sur le bloc de blindage (13).

14. Collimateur selon la revendication 13, **caractérisé en ce que** l'autre bloc de blindage (17) est disposé fixe sur le collimateur (1) et son extension est mesurée de sorte que les ouvertures de collimateur (5, 5'; 5' ou 5. 5") n'étant pas en la position de travail se trouvent dans l'opacité de l'autre bloc de blindage (17) à chaque déplacement de l'ouverture de collimateur (5", 5 ou 5') se trouvant en position de travail par rapport aux rayons (2).

15. Collimateur selon l'une des revendications 1 à 14, **caractérisé en ce que** la trajectoire de surface sphérique (6) est formée par deux glissières (18, 19) s'étendant verticalement entre elles, sur lesquelles coulisse le boc de blindage (13).

16. Collimateur selon la revendication 15, **caractérisé en ce que** les glissières (18, 19) sont formées par des paires de glissières (18', 18", 19', 19"), une première paire de glissières (18', 18") étant reliée au coffret de collimateur (22) pouvant être disposé sur l'appareil de radiothérapie, la seconde paire de glissière (19', 19") étant associée à un premier chariot de glissière (20) qui court sur la première paire de glissière (18', 18") et que le bloc de blindage est disposé sur un second chariot de glissière (21) qui court sur la second paire de glissières (19', 19").

17. Collimateur selon l'une des revendications 1 à 16, **caractérisé en ce que** le dispositif d'entraînement (8) pour le déplacement d'au moins une ouverture de collimateur (5, 5', 5") sur la trajectoire de surface sphérique (6) présente deux entraînements (23, 24).

18. Collimateur selon la revendication 16 et 17, **caractérisé en ce que** les entraînements (23, 24) sont disposés sur le chariot coulissant (20, 21) pour le déplacement de ce dernier.

19. Collimateur selon la revendication 17 ou 18, **caractérisé en ce que** les entraînements (23, 24) sont munis de broches filetées sphériques.

20. Collimateur selon l'une des revendications 1 à 20, **caractérisé en ce que** les dispositifs d'entraînement (23, 24) sont munis de broches filetées horizontales.

21. Collimateur selon l'une des revendications 1 à 20, **caractérisé en ce que** les dispositifs d'entraînement (8) présentent un autre entraînement (25) pour amener l'ouverture de collimateur souhaitée (5, 5' ou 5") en position de travail.

22. Collimateur selon la revendication 21 , **caractérisé en ce que** l'autre entraînement (25) est disposé sur le bloc de blindage (13) pour tourner la partie cylindrique (12) dans les positions de travail des ouvertures de collimateur (5, 5' ou 5").

23. Collimateur selon l'une des revendications 1 à 22, **caractérisé en ce que** le dispositif d'entraînement (8) présente au moins un entraînement de moteur électrique (23, 24, 25) .

24. Collimateur selon l'une des revendications 1 à 23, **caractérisé en ce que** le dispositif moteur (8) présente au moins un entraînement pneumatique (23, 24, 25).

25. Collimateur selon l'une des revendications 1 à 24, **caractérisé en ce que** le dispositif d'entraînement (8) présente au moins un entraînement hydraulique (23, 24, 25).

26. Collimateur selon l'une des revendications 1 à 25, **caractérisé en ce que** la commande (9) est réalisée de sorte que le temps de séjour des rayons (2) peut être commandé au moyen du dispositif d'entraînement (8, 23, 24).

27. Collimateur selon la revendication 26, **caractérisé en ce que** la commande (9) est réalisée de sorte que le temps de séjour peut être obtenu par la vitesse de balayage des rayons (2') à laquelle ces derniers balayent l'objet de traitement (4).

28. Collimateur selon l'une des revendications 1 à 27, **caractérisé en ce que** la commande (9) est un calculateur comportant un programme correspondant.

29. Collimateur selon l'une des revendications 1 à 28, **caractérisé en ce qu'**il est muni de dispositifs de saisie de position pour les mouvements de réglage.

30. Collimateur selon l'une des revendications 1 à 29, **caractérisé en ce que** la commande (9) est réalisée de sorte que l'opération de balayage s'effectue au moyen d'un plan thérapeutique prédéterminé.

31. Collimateur selon la revendication 29 et 30, **caractérisé en ce que** la commande (9) est reliée aux dispositifs de saisie de position et est réalisée de sorte que les saisies de position servent à la vérification et à la correction des mouvements de réglage.

32. Programme pour la commande d'un collimateur selon l'une des revendications 1 à 31, **caractérisé en ce qu'**il est conçu de sorte qu'il commande au moyen d'un ordinateur le dispositif d'entraînement (8, 23, 24) de sorte qu'un objet de traitement (4) peut être irradié en fonction des contours prédéfinis (29, 29', 29"), le programme sélectionnant par actionnement de l'autre entraînement (25) la dimension optimale d'une ouverture de collimateur (5, 5' ou 5") pour la réalisation du contour (29, 29', 29") et les opérations de balayage qui sont exécutées par les différentes ouvertures de collimateur (5, 5', 5") sont synchronisées entre elles pour atteindre l'irradiation exigée.

33. Programme selon la revendication 32, **caractérisé en ce qu'**il est réalisé de sorte qu'il peut varier l'intensité d'irradiation (30, 30', 30") au moyen du temps de séjour par une commande correspondante du dispositif d'entraînement (8, 23, 24).

34. Programme selon la revendication 32 ou 33, **caractérisé en ce qu'**il est réalisé de sorte qu'il divise la surface d'irradiation respective (26) pour un objet de traitement (4) en segments de champ partiel (28. 28', 28") et commande l'irradiation au moyen de l'ordinateur selon ces segments de champs partiel (28, 28', 28").

35. Programme selon la revendication 34, **caractérisé en ce qu'**il procède à la division en segments de champs partiel (28, 28', 28") pour les surfaces d'irradiation (26) pour différents directions d'irradiation (27) à partir desquels doit être irradié l'objet de traitement (4).

36. Programme selon l'une des revendications 32 à 35, **caractérisé en ce qu'**il est réalisé de sorte qu'il définit la détermination des surfaces d'irradiation (26) sur la base de la détection des contours (29, 29', 29") par une saisie d'image de l'objet de traitement (4).

37. Programme selon la revendication 36, **caractérisé en ce qu'**il définit également les surfaces d'irradiation (26) avec des intensités différentes d'irradiation (30, 30', 30") sur la base de contours (29, 29', 29") de différentes zones partielles de l'objet d'irradiation (4).

38. Programme selon l'une des revendications 32 à 37, **caractérisé en ce qu'**il est conçu de sorte qu'il assume la commande du dispositif moteur (8, 23, 24) sur la base d'un traitement de la détection d'image par un plan de traitement.
